# EUROPÄISCHE PATENTANMELDUNG

(11) **EP 0 744 175 A2**
(43) Veröffentlichungstag der Anmeldung: **27.11.1996**
(21) Anmeldenummer: 96108038.9
(22) Anmeldetag: 21.05.1996
(51) Int. Cl.: A61K 7/48, A61K 31/20

(54) **Kosmetische oder dermatologische Zubereitungen mit einem Gehalt an alpha-Hydroxyfettsäuren**

(30) Priorität: 23.05.1995 DE 19518815
(71) Anmelder: Beiersdorf Aktiengesellschaft, D-20245 Hamburg (DE)
(72) Erfinder: Schreiner, Volker, Dr., 20259 Hamburg (DE); Schmucker, Robert, Dr., 22457 Hamburg (DE); Stäb, Franz, Dr., 21379 Echem (DE); Sauermann, Gerhard, Dr., 24649 Wiemersdorf (DE); Paal, Michael, Dr., 22335 Hamburg (DE); Wolf, Florian, Dr., 20251 Hamburg (DE)
(74) Vertreter: Dinné, Erlend

(57) **Zusammenfassung**

Gegenstand der Erfindung ist die Verwendung von einer alpha-Hydroxyfettsäure oder mehreren alpha-Hydroxyfettsäuren oder Salzen davon zur Prophylaxe und Behandlung von schädigenden oxidativen Einwirkungen auf die Haut oder die Haare.

## Beschreibung

Die Erfindung betrifft Wirkstoffe und kosmetische oder dermatologische Zubereitungen, insbesondere topische Zubereitungen, die die Folgeschäden oxidierend wirkender Substanzen oder einer akuten oder chronischen Lichtexposition, insbesondere einer Ultraviolettlichtexposition, lindern und die vor den Folgeschäden oxidierend wirkender Substanzen oder einer akuten oder chronischen Licht-UV-Exposition schützen, also präventiv wirken.

Die schädigenden Effekte des Sonnenlichtes sind gut beschrieben. Dabei verursachen Strahlen im Bereich zwischen 290 nm und 320 nm, dem sogenannten UVB-Bereich, akut bei Überschreitung einer minimalen Dosis ein Erythem, einen einfachen Sonnenbrand. Als ein Maximum der Erythemwirksamkeit wird der engere Bereich um 308 nm angegeben.

Über diesen Akutschaden hinaus kann Sonnenlicht, insbesondere im UVA-Bereich von 320 nm bis 400 nm, bei wiederholter, also chronischer Exposition kumulativ schädigend wirksam sein und eine Schädigung der elastischen und kollagenen Fasern der Haut verursachen. Dieser Prozeß wird als Photoalterung der Haut bezeichnet. Er überlagert und beschleunigt den natürlichen Alterungsprozess der Haut und bedingt deren zunehmende Schlaffheit und Faltigkeit. Darüberhinaus gehören die sichtbare Gefäßerweiterung (Couperosis), lokale Fehlpigmentierungen (z.B. Altersflecken), die vergrößerte Anfälligkeit gegenüber mechanischem Streß (z.B. Rissigkeit) zum Symptomkomplex der photogealterten, also chronisch UVA-exponierten Haut. Alle diese Phänomene sind detailliert im Handbook of Nonprescription Drugs, 7th Ed. Chapter 26, pp: 499-511 (Am. Pharmaceutical Association, Washington, D.C., 1982) beschrieben.

UVA-Strahlung ist auch die Ursache zahlreicher phototoxischer und photoallergischer Reaktionen. Ferner kann sie den schädigenden Einfluß der UVB-Strahlung verstärken.

Letztlich liegt ein großer Teil der Ursachen für die zuvor aufgeführten makroskopischen Phänomene der Photoalterung in Funktionsstörungen der Hautzellen begründet, deren enzym- also proteinkatalysierte, biochemische Aktivität (Hautmetabolismus) durch den Photoalterungsprozess signifikant eingeschränkt ist. Zum anderen sind von der Schädigung durch chronische UV-Exposition Gerüst- und Strukturproteine (z.B. Keratine, Mikrofilamente) in- und außerhalb der Zellen betroffen, die beispielsweise die mechanischen Eigenschaften des Zellverbandes Haut ausmachen. Dabei kann die UV-Strahlung zu photochemischen Reaktionen führen, wobei die photochemischen Reaktionsprodukte in alle Ebenen des Hautmetabolismus eingreifen.

Eine Ursache des endogenen und durch äußere Einflüsse beschleunigten Hautalterungsprozesses ist eine Zunahme des Gehaltes an Ketoproteinen in der Hornschicht. Ketoproteine entstehen durch eine oxidative Modifikation von Seitengruppen von Aminosäureresten des Proteins. In diese Seitenketten wird eine Carbonylgruppe unter Mitwirkung von starken Oxidationsmitteln (z.B. H₂O₂) eingeführt. Ketoproteine dienen aber auch als Marker für den UV- bzw. Sauerstoffradikal-induzierten Schaden an Proteinen der Epidermis.

Auch menschliches Haar ist dem schädigenden Effekten des UV-Lichtes ausgesetzt und verwittert im Verlauf seiner Alterung u.a. durch oxidative Aufspaltung von stabilisierenden Disulfidbrücken. Weiterhin werden bei einer Vielzahl von Haarfärbeprozessen hohe Konzentrationen an Wasserstoffperoxiden eingesetzt, die möglicherweise als Nebenwirkung eine Schädigung der Haarsubstanz bedingen können.

Vorwiegend handelt es sich bei den photochemischen Reaktionsprodukten um radikalische Verbindungen, beispielsweise Hydroxyradikale. Auch undefinierte radikalische Photoprodukte, welche in der Haut und Haaren selbst entstehen, können aufgrund ihrer hohen Reaktivität unkontrollierte Folgereaktionen initiieren. Aber auch Singulettsauerstoff, ein nichtradikalischer, angeregter und sehr reaktiver Zustand des Sauerstoffmoleküls kann bei UV-Bestrahlung auftreten, ebenso kurzlebige Epoxide und viele andere.

Ferner zählt UV-Strahlung zur ionisierenden Strahlung. Es besteht also das Risiko, daß auch ionische Spezies bei UV-Exposition entstehen, welche dann ihrerseits z.B. oxidativ in biochemische Prozesse der Haut eingreifen können.

Um diesen Reaktionen vorzubeugen und den Schutz der Haut vor UV-Strahlung zu gewährleisten, werden zahlreiche Verbindungen in topischen Präparaten eingesetzt, die die UVB- und/oder UVA-Strahlung absorbieren, also filtern.

Typische UVB-Filter sind Derivate des 3-Benzylidencamphers, der 4-Aminobenzoesäure, der Zimtsäure, der Salicylsäure, des Benzophenons sowie des 2-Phenylbenzimidazols. Zum Schutz der Haut vor UVA-Strahlung kommen zumeist gewisse Derivate des Dibenzoylmethans zum Einsatz.

UV-Filter bergen jedoch folgende Probleme für den Verbraucher in sich: Sie entfalten lediglich einen schützenden, jedoch keinen therapeutischen Effekt. Um einen optimalen Schutz zu gewährleisten, müssen die Filtersubstanzen die Haut dauerhaft mit einen gleichmäßigen und gleichverteilten Film überziehen.

Diese Voraussetzungen sind jedoch kaum zu erfüllen, da die Hautoberfläche von Fältchen durchsetzt ist und der Anwender sich bei Sonnenlicht häufig auch im Wasser bewegt bzw. schwitzt. Darüberhinaus können UV-Filter ein gewisses Irritationsrisiko für Schleimhäute in sich bergen und gelegentlich ein Brenngefühl an empfindlichen Hautpartien verursachen (Stinging-Potential).

Als weiterer, rein physikalischer Schutz werden topischen Zubereitungen UV-reflektierende bzw. UV-streuende Substanzen beigemischt. Stoffe wie Titandioxid bzw. Zinkoxid erfüllen diese Aufgabe, verleihen den Zubereitungen jedoch eine unattraktive, weißliche Farbe. Außerdem gelingt es nicht immer, gerade diese Substanzen auf der Hautoberfläche ausreichend zu fixieren.

Um den photochemischen Reaktionen vorzubeugen, können kosmetischen bzw. dermatologischen Zubereitungen auch Antioxidantien und/oder Radikalfänger zugesetzt werden. Antioxidantien sind Substanzen, welche Oxidationsprozesse in dem sie umgebenden Medium (Haut, Haare oder Produkt) verhindern. Antioxidantien, welche auf den Gebieten der Kosmetik und Pharmazie Verwendung finden, sind beispielsweise α-Tocopherol, insbesondere in Form des α-Tocopherolacetats, Butylhydroxyanisols und Butylhydroxytoluol.

In den US-Patentschriften 4.144.325 und 4.248.861 sowie zahlreichen anderen Dokumenten ist vorgeschlagen worden, Vitamin E, eine Substanz mit bekannter antioxidativer, sprich sauerstoffradikalneutralisierenden Wirkung in Lichtschutzformulierungen einzusetzen, dennoch bleibt auch hier die erzielte Wirkung hinter der erhofften zurück.

Eine Aufgabe der vorliegenden Erfindung war es, die Nachteile des Standes der Technik zu beseitigen. Insbesondere sollte ein Wirkstoff bzw. Zubereitungen, diesen Wirkstoff enthaltend, zur Verfügung gestellt werden, bei deren Verwendung die Schädigungen der Haut und/oder Haare durch oxidativen Einfluß gemindert oder verringert werden können, z.B. indem die Ketoproteinbildung verringert wird.

Eine weitere Aufgabe der vorliegenden Erfindung bestand darin, insbesondere kosmetische Zubereitungen zur Verfügung zu stellen, welche der Haaralterung oder vor oder nach Behandlung des Haares mit Bleichmitteln oder Haarfärbezubereitungen, selbst solcher mit einem hohen Gehalt an starken Oxidationsmitteln, z.B. Wasserstoffperoxid, deren schädigender Oxidationswirkung entgegenwirken.

Diese Aufgaben werden gemäß der vorliegenden Erfindung gelöst.

Gegenstand der Erfindung ist die Verwendung von einer alpha-Hydroxyfettsäure oder mehreren alpha-Hydroxyfettsäuren oder Salzen davon zur Prophylaxe und Behandlung von schädigenden oxidativen Einwirkungen auf die Haut oder die Haare.

Gegenstand der Erfindung ist auch die Verwendung von kosmetischen oder dermatologischen Zubereitungen mit einem Gehalt an einer alpha-Hydroxyfettsäure oder mehreren alpha-Hydroxyfettsäuren oder Salzen davon zur Prophylaxe und Behandlung von schädigenden oxidativen Einwirkungen auf die Haut oder die Haare.

Bevorzugt werden topische Zubereitungen.

Gemische können von zwei, mehreren aber auch einer größeren Anzahl von Verbindungen gebildet werden und diese Verbindungen in unterschiedlichen Gewichtsmengen enthalten. So können aus natürlichen Quellen stammende Fraktionen eine größere Zahl aufgrund von Homologen oder Strukturisomeren aufweisen, synthetisierte Säuren können dagegen aus jeweils einer Verbindung bestehen. Erfindungsgemäß soll mindestens eine der genannten alpha-Hydroxysäuren verwendet werden oder in der Zubereitung enthalten sein.

Die erfindungsgemäßen alpha-Hydroxyfettsäuren können geradkettig oder verzweigt sein. Bevorzugt werden die gesättigten, aliphatischen alpha-Hydroxyfettsäuren.

Bevorzugte erfindungsgemäße alpha-Hydroxyfettsäuren enthalten 8 bis 20 Kohlenstoffatome, insbesondere aber 14 bis 18 Kohlenstoffatome, besonders bevorzugt 16 Kohlenstoffatome. Vorteilhaft sind auch Gemische der in solchen Bereichen liegenden Fettsäuren.

Besonders bevorzugt sind die folgenden alpha-Hydroxyfettsäuren und ihre Salze, insbesondere in der D-Form:
alpha-Hydroxy-hexadecansäure
(alpha-Hydroxy-palmitinsäure).

Erfindungsgemäß können auch aus Wollwachs gewonnene alpha-Hydroxyfettsäuren eingesetzt werden, z.B. gemäß bekannten Isolierungsverfahren erhältliche alpha-Hydroxy-Fettsäuregemische (DE-A-42 04 321) die aus Wollwachssäuregemischen gewonnen werden. Sie fallen vorzugsweise als D-Enantiomeren-Gemisch an. Auch die nach bekannten Verfahren erhältlichen Einzelverbindungen sind bevorzugt.

Die erfindungsgemäßen alpha-Hydroxyfettsäuren sind bekannt oder nach bekannten Verfahren erhältlich. In der Synthese anfallende Racemate lassen sich in üblichen Verfahren in die Isomeren aufspalten.

Bevorzugte Salze sind verträgliche Salze, insbesondere wasserlösliche Salze, z.B. die Natrium- oder Kaliumsalze. Vorzugsweise werden die erfindungsgemäßen Wirkstoffe in der Säureform eingesetzt.

α-Hydroxyfettsäuren sind natürliche Bestandteile der in der menschlichen Hornschicht vorhandenen Lipidmembranen. Sie sind hier amidartig an das Sphingosin der Ceramide 4,5 und 6I bzw. 6II gebunden und können durch ein in der Hornschicht vorhandenes Enzym, die Ceramidase, freigesetzt werden. Sie besitzen keine UV-absorbierenden Eigenschaften und sind somit nicht den Filtersubstanzen zuzurechnen.

Als besonders vorteilhafte α-Hydroxyfettsäure im Sinne der vorliegenden Erfindung wird die α-Hydroxypalmitinsäure angesehen. Die α-Hydroxypalmitinsäure ist ein natürlicher Bestandteil der Wollwachsäuren des Schafes, womit zugleich eine Quelle dieses Wirkstoffes für kosmetische oder dermatologische Zubereitungen bezeichnet ist.

Die Offenlegungsschrift DE-A- 4204321 beschreibt zwar die desodorierenden Eigenschaften, die Fähigkeit als Konservierungshelfer zu dienen oder Ionen zu binden. Es war aber für den Fachmann nicht vorauszusehen oder der Schrift zu entnehmen, das die erfindungsgemäße Wirkstoffgruppe die erfindungsgemäßen Eigenschaften entfalten würde.

Unter dem Begriff 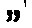 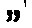 oxidativer Einwirkung" ist sowohl die Wirkung von oxidierend wirkenden Substanzen zu verstehen als auch die oxidative Wirkung von Strahlung, namentlich Licht, insbesondere UV-Licht und den dadurch hervorgerufenen Folgeprodukten.

Mit 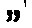 schädigend" sind solche Einwirkungen gemeint, die unerwünschte Veränderungen der Haut oder der Haare hervorrufen, z.B. die Alterung, oder Ketoproteinbildung, aber z.B. nicht die gewünschte Bleichwirkung von Haarbleichmitteln oder z.B. die gewünschte Wirkung von Haarfarben.

Die erfindungsgemäßen Wirkstoffe und Zubereitungen zeichnen sich dadurch aus, daß sie die Folgeschäden oxidierend wirkender Substanzen oder einer akuten oder chronischen Lichtexposition, insbesondere einer Ultraviolettlichtexposition, lindern und vor den Folgeschäden oxidierend wirkender Substanzen oder einer akuten oder chronischen Lichtexposition oder UV-Exposition schützen, also präventiv wirken.

Überraschenderweise wurde auch gefunden, daß die erfindungsgemäßen Wirkstoffe und Zubereitungen die Bildung von Ketoproteinen in der Haut reduzieren und die Hautveränderungen oder Hautschäden, die mit der Hautalterung, insbesondere der endogenen Hautalterung und der Photoalterung der Haut auftreten, verringern oder verhindern. Sie wirken dabei präventiv, aber sie können auch bereits entstandene Schäden beheben.

Besonders bevorzugt ist die Verwendung der erfindungsgemäßen Wirkstoffe und Zubereitungen zur Prophylaxe und Behandlung von Hautveränderungen und Hautschäden der Hautalterung, insbesondere der endogenen Hautalterung und der Photoalterung der Haut und der Ketoproteinbildung.

Bei der Photoalterung können die erfindungsgemäßen Wirkstoffe und Zubereitungen bei UVA- und/oder UVB-Bestrahlung verwendet werden.

Auch die Haare unterliegen einem ständigen Alterungsprozeß, der überwiegend auf oxidativen Einwirkungen beruht.

Soll menschliches Haar dauerhaft gefärbt werden, kommen in der Praxis lediglich oxidierende Haarfärbeverfahren in Betracht. Beim oxidativen Haarfärben erfolgt die Ausbildung des Farbstoffchromophoren durch Reaktion von Präkursoren (Phenole, Aminophenole, seltener auch Diamine) und Basen (meistens p-Phenylendiamin) mit dem Oxidationsmittel, zumeist Wasserstoffperoxid. Wasserstoffperoxidkonzentrationen um 6% werden dabei gewöhnlich verwendet.

Üblicherweise wird davon ausgegangen, daß neben der Färbewirkung auch eine Bleichwirkung durch das Wasserstoffperoxid erfolgt. In oxidativ gefärbtem menschlichem Haar sind, ähnlich wie bei gebleichtem Haar, mikroskopische Löcher an den Stellen, an denen Melaningranula vorlagen, nachweisbar.

Es ist nicht auszuschließen, daß das Oxidationsmittel Wasserstoffperoxid nicht nur mit den Farbvorstufen, sondern auch mit der Haarsubstanz reagieren und dabei unter Umständen eine Schädigung des Haares bewirken kann.

Auch zum Bleichen von Haaren und beim Formen der Haare werden oxidativ wirkende Mittel verwendet, die Schäden bewirken können.

Gegenstand der Erfindung ist insbesondere auch die Verwendung der erfindungsgemäßen Wirkstoffe und Zubereitungen zur Prophylaxe und Behandlung von Haarschäden durch Haarkosmetika, z.B. Bleichmittel, Färbemittel und formgebende Mittel.

Es war für den Fachmann daher nicht vorauszusehen gewesen, daß die erfindungsgemäßen Wirkstoffe bzw. Zubereitungen, diese enthaltend
- besser reduzierend auf die Entstehung von Radikalen wirken
- besser die Bindung von schädlichen Photoprodukten an Lipide, DNS und Proteine verhindern
- besser die Ketoproteinbildung in der Haut reduzieren
- besser gegen die Hautalterung wirken
- besser die Haut gegen Photoreaktionen schützen
- besser auf die Haut aufziehen
als die Wirkstoffe, Wirkstoffkombinationen und Zubereitungen des Standes der Technik.

Die erfindungsgemäßen kosmetischen oder dermatologischen Zubereitungen oder Formulierungen können wie üblich zusammengesetzt sein und zur Behandlung, der Pflege und der Reinigung der Haut und/oder der Haare und als Schminkprodukt in der dekorativen Kosmetik dienen. Sie enthalten z.B. 0,001 bis 10 Gew.-%, bevorzugt 0,1 Gew.-% bis 1 Gew.-%, insbesondere aber 0,1 Gew.-% bis 0,5 Gew.-%, jeweils bezogen auf das Gesamtgewicht der Zubereitungen an den erfindungsgemäßen Wirkstoffen.

Zubereitungen zur Prophylaxe, Behandlung und Pflege der Haut werden besonders bevorzugt.

Zur Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z.B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsionen, beispielsweise vom Typ Wasser-in-Öl-in-Wasser (W/O/W), ein Gel, einen festen Stift, eine Salbe oder auch ein Aerosol darstellen. Es ist auch vorteilhaft, die erfindungsgemäßen Wirkstoffe in verkapselter Form darzureichen, z.B. in Kollagenmatrices und anderen üblichen Verkapselungsmaterialien, z.B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt.

Es ist auch möglich und vorteilhaft im Sinne der vorliegenden Erfindung, die erfindungsgemäßen Wirkstoffe in wäßrige Systeme bzw. Tensidzubereitungen zur Reinigung der Haut und der Haare einzufügen.

Als vorteilhafte Verkörperung der vorliegenden Erfindung wird daher auch die Verwendung erfindungsgemäßer Wirkstoffe zum Schutze der Haut und/oder der Haare vor oxidativer Schädigung angesehen, insbesondere diese Verwendung der erfindungsgemäßen Wirkstoffe in Shampoos und Waschformulierungen.

Die erfindungsgemäßen kosmetischen und dermatologischen Zubereitungen können kosmetische Hilfsstoffe enthalten, wie sie üblicherweise in solchen Zubereitungen verwendet werden, z.B. Konservierungsmittel, Bakterizide, Parfüme, Substanzen zum Verhindern des Schäumens, Farbstoffe, Pigmente, die eine färbende Wirkung haben, Verdickungsmittel, oberflächenaktive Substanzen, Emulgatoren, weichmachende, anfeuchtende und/oder feuchhaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Insbesondere können die erfindungsgemäßen Wirkstoffe für die erfindungsgemäßen Wirkungen auch mit Antioxidantien kombiniert werden. Auch solche Kombinationen sind Gegenstand der Erfindung wie auch die Verwendungen, wie sie schon für die Wirkstoffe angegeben wurden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Sesamol, Sesamolin, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die Lipidphase kann dabei vorteilhaft gewählt werden aus folgender Substanzgruppe:
- natürliche, synthetische und/oder partialsynthetische Öle, wie Triglyceride der Caprin- oder der Caprylsäure, vorzugsweise aber Rizinusöl;
- Fette, Wachse und andere natürliche synthetische und/oder partialsynthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.
- gesättigte Verbindungen wie Kohlenwasserstoffe natürlichen oder synthetischen Ursprungs (Vaseline, Squalan)

Die wäßrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, oder auch der Typen ETD (Easy-to-disperse) 2001, 2020, 2050, jeweils einzeln oder in beliebigen Kombinationen untereinander.Kombination.

Insbesondere werden Gemische der vorstehend genannten Lösemittel verwendet. Bei alkoholischen Lösemitteln kann Wasser ein weiterer Bestandteil sein.

Erfindungsgemäße Emulsionen sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei öligalkoholischen Gelen vorzugsweise Siliciumdioxid oder ein Aluminiumsilikat, bei wäßrig-alkoholischen oder alkoholischen Gelen vorzugweise ein Polyacrylat ist.

Als Treibmittel für erfindungsgemäße, aus Aerosolbehältern versprühbare Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Vorteilhaft können erfindungsgemäße Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

Enthalten die erfindungsgemäßen Emulsionen UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoësäure-Derivate, vorzugsweise 4-(Dimethylamino)-benzoësäure(2-ethylhexyl)ester, 4-(Dimethylamino)benzoësäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester, - 2,4,6-Trianilino-(p-carbo-2'-ethyl-1'-hexyloxy) -1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Die Liste der genannten UVB-Filter, die in Kombination mit den erfindungsgemäßen Wirkstoffen verwendet werden können, soll selbstverständlich nicht limitierend sein.

Gegenstand der Erfindung ist auch die Verwendung der erfindungsgemäßen Wirkstoffe mit mindestens einem UVB-Filter als Antioxidans bzw. die Verwendung der erfindungsgemäßen Wirkstoffe mit mindestens einem UVB-Filter in einer kosmetischen oder dermatologischen Zubereitung.

Es kann auch von Vorteil sein, erfindungsgemäße Wirkstoffe mit UVA-Filtern zu kombinieren. Beispielsweise sind solche geeignet, die bisher üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)propan-1,3-dion und um 1-Phenyl-3-(4'-isopropylphenyl)propan-1,3-dion. Auch diese Kombinationen bzw. Zubereitungen, die diese Kombinationen enthalten, sind Gegenstand der Erfindung. Es können die für die UVB-Kombination verwendeten Mengen eingesetzt werden.

Gegenstand der Erfindung ist auch die Verwendung einer Kombination der erfindungsgemäßen Wirkstoffe mit mindestens einem UVA-Filter bzw. die Verwendung einer Kombination der erfindungsgemäßen Wirkstoffe mit mindestens einem UVA-Filter in einer kosmetischen oder dermatologischen Zubereitung.

Gegenstand der Erfindung ist auch die Verwendung einer Kombination der erfindungsgemäßen Wirkstoffe mit mindestens einem UVA-Filter und mindestens einem UVB-Filter bzw. die Verwendung einer Kombination der erfindungsgemäßen Wirkstoffe mit mindestens einem UVA-Filter und mindestens einem UVB-Filter in einer kosmetischen oder dermatologischen Zubereitung.

Kosmetische und dermatologische Zubereitungen mit einem wirksamen Gehalt an erfindungsgemäßen Wirkstoffen können auch anorganische Pigmente enthalten, die üblicherweise in der Kosmetik zum Schutze der Haut vor UV-Strahlen verwendet werden. Dabei handelt es sich um Oxide des Titans, Zinks, Eisens, Zirkoniums, Siliciums, Mangans, Aluminiums, Cers und Mischungen davon, sowie Abwandlungen, bei denen die Oxide die aktiven Agenten sind. Besonders bevorzugt handelt es sich um Pigmente auf der Basis von Titandioxid.

Auch diese Kombinationen von UVA-Filter und Pigment bzw. Zubereitungen, die diese Kombination enthalten, sind Gegenstand der Erfindung. Es können die für die vorstehenden Kombinationen genannten Mengen verwendet werden.

Bei kosmetischen und dermatologischen Zubereitungen zum Schutze der Haare vor UV-Strahlen gemäß der Erfindung handelt es sich beispielsweise um Shampoonierungsmittel, Zubereitungen, die beim Spülen der Haare vor oder nach der Shampoonierung, vor oder nach der Dauerwellbehandlung, vor oder nach der Färbung oder Entfärbung der Haare angewendet werden, um Zubereitungen zum Fönen oder Einlegen der Haare, Zubereitungen zum Färben oder Entfärben, um eine Frisier- und Behandlungslotion, einen Haarlack oder um Dauerwellmittel.

Die Kombinationen mit UV-Filtern und UV-Schutzstoffen haben die für die Wirkstoffe beschriebenen Wirkungen und können in gleicher Weise verwendet werden.

Die kosmetischen und dermatologischen Zubereitungen enthalten Wirkstoffe und Hilfsstoffe, wie sie üblicherweise für diesen Typ von Zubereitungen zur Haarpflege und Haarbehandlung verwendet werden. Als Hilfsstoffe dienen Konservierungsmittel, oberflächenaktive Substanzen, Substanzen zum Verhindern des Schäumens, Verdickungsmittel, Emulgatoren, Fette, Öle, Wachse, organische Lösungsmittel, Bakterizide, Parfüme, Farbstoffe oder Pigmente, deren Aufgabe es ist, die Haare oder die kosmetische oder dermatologische Zubereitung selbst zu färben, Elektroyte, Substanzen gegen das Fetten der Haare.

Unter Elektrolyten im Sinne der vorliegenden Erfindung sind wasserlösliche Alkali-, Ammonium-, Erdalkali- (unter Einbeziehung des Magnesiums) und Zinksalze anorganischer Anionen und beliebige Gemische aus solchen Salzen zu verstehen, wobei gewährleistet sein muß, daß sich diese Salze durch pharmazeutische oder kosmetische Unbedenklichkeit auszeichnen.

Die erfindungsgemäßen Anionen werden bevorzugt gewählt aus der Gruppe der Chloride, der Sulfate und Hydrogensulfate, der Phosphate, Hydrogenphosphate und der linearen und cyclischen Oligophosphate sowie der Carbonate und Hydrogencarbonate.

Kosmetische Zubereitungen, die ein Hautreinigungsmittel oder Shampoonierungsmittel darstellen, enthalten vorzugsweise mindestens eine anionische, nicht-ionische oder amphotere oberflächenaktive Substanz, oder auch Gemische aus solchen Substanzen, erfindungsgemäße Wirkstoffkombinationen im wäßrigen Medium und Hilfsmittel, wie sie üblicherweise dafür verwendet werden. Die oberflächenaktive Substanz bzw. die Gemische aus diesen Substanzen können in einer Konzentration zwischen 1 Gew.-% und 50 Gew.-% in dem Shampoonierungsmittel vorliegen.

Liegen die kosmetischen oder dermatologischen Zubereitungen in Form einer Lotion vor, die ausgespült und z.B. vor oder nach der Entfärbung, vor oder nach der Shampoonierung, zwischen zwei Shampoonierungsschritten, vor oder nach der Dauerwellbehandlung angewendet wird, so handelt es sich dabei z.B. um wäßrige oder wäßrig-alkoholische Lösungen, die gegebenenfalls oberflächenaktive Substanzen enthalten, deren Konzentration zwischen 0,1 und 10 Gew.-%, vorzugsweise zwischen 0,2 und 5 Gew.-%, liegen kann.

Diese kosmetischen oder dermatologischen Zubereitungen können auch Aerosole mit den üblicherweise dafür verwendeten Hilfsmitteln darstellen.

Eine kosmetische Zubereitung in Form einer Lotion, die nicht ausgespült wird, insbesondere eine Lotion zum Einlegen der Haare, eine Lotion, die beim Fönen der Haare verwendet wird, eine Frisier- und Behandlungslotion, stellt im allgemeinen eine wäßrige, alkoholische oder wäßrig-alkoholische Lösung dar und enthält mindestens ein kationisches, anionisches, nicht-ionisches oder amphoteres Polymer oder auch Gemische derselben, sowie erfindungsgemäße Wirkstoffkombinatonen in wirksamer Konzentration. Die Menge der verwendeten Polymeren liegt z.B. zwischen 0,1 und 10 Gew.-%, bevorzugt zwischen 0,1 und 3 Gew.-%.

Kosmetische Zubereitungen zur Behandlung und Pflege der Haare, die erfindungsgemäße Wirkstoffe enthalten, können als Emulsionen vorliegen, die vom nicht-ionischen oder anionischen Typ sind. Nicht-ionische Emulsionen enthalten neben Wasser Öle oder Fettalkohole, die beispielsweise auch polyethoxyliert oder polypropoxyliert sein können, oder auch Gemische aus den beiden organischen Komponenten. Diese Emulsionen enthalten gegebenenfalls kationische oberflächenaktive Substanzen.

Erfindungsgemäß können kosmetische Zubereitungen zur Behandlung und Pflege der Haare als Gele vorliegen, die neben einem wirksamen Gehalt an erfindungsgemäßen Wirkstoffkombinationen und dafür üblicherweise verwendeten Lösungsmitteln, bevorzugt Wasser, noch organische Verdickungsmittel, z.B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel` z.B. Aluminiumsilikate wie beispielsweise Bentonite, oder ein Gemisch aus Polyethylenglykol und Polyethylenglycolstearat oder -distearat, enthalten. Das Verdickungsmittel ist in dem Gel z.B. in einer Menge zwischen 0,1 und 30 Gew.-%, bevorzugt zwischen 0,5 und 15 Gew.-%, enthalten.

Vorzugsweise beträgt die Menge der erfindungsgemäßen Wirkstoffe in einem für die Haare bestimmten Mittel 0,05 Gew.-% bis 10 Gew.-%, insbesondere 0,5 Gew.-% bis 5 Gew.-%, bezogen auf das Gesamtgewicht des Mittels.

Erfindungsgemäße wäßrige kosmetische Reinigungsmittel oder für die wäßrige Reinigung bestimmte wasserarme oder wasserfreie Reinigungsmittelkonzentrate können anionische, nichtionische und/oder amphotere Tenside enthalten, beispielsweise
- herkömmliche Seifen, z.B. Fettsäuresalze des Natriums
- Alkylsulfate, Alkylethersulfate, Alkan- und Alkylbenzolsulfonate
- Sulfoacetate
- Sulfobetaine
- Sarcosinate
- Amidosulfobetaine
- Sulfosuccinate
- Sulfobernsteinsäurehalbester
- Alkylethercarboxylate
- Eiweiß-Fettsäure-Kondensate
- Alkylbetaine und Amidobetaine
- Fettsäurealkanolamide
- Polyglycolether-Derivate
Kosmetische Zubereitungen, die kosmetische Reinigungszubereitungen für die Haut darstellen, können in flüssiger oder fester Form vorliegen. Sie enthalten neben erfindungsgemäßen Wirkstoffkombinationen vorzugsweise mindestens eine anionische, nicht-ionische oder amphotere oberflächenaktive Substanz oder Gemische daraus, gewünschtenfalls einen oder mehrere Elektrolyten und Hilfsmittel, wie sie üblicherweise dafür verwendet werden. Die oberflächenaktive Substanz kann in einer Konzentration zwischen 1 und 94 Gew.-% in den Reinigungszubereitungen vorliegen, bezogen auf das Gesamtgewicht der Zubereitungen.

Kosmetische Zubereitungen, die ein Shampoonierungsmittel darstellen, enthalten neben einem wirksamen Gehalt an erfindungsgemäßen Wirkstoffen vorzugsweise mindestens eine anionische, nicht-ionische oder amphotere oberflächenaktive Substanz oder Gemische daraus, gegebenenfalls einen erfindungsgemäßes Elektrolyten und Hilfsmittel, wie sie üblicherweise dafür verwendet werden. Die oberflächenaktive Substanz kann in einer Konzentration zwischen 1 Gew.-% und 94 Gew.-% in dem Shampoonierungsmittel vorliegen.

Die erfindungsgemäßen Zusammensetzungen enthalten außer den vorgenannten Tensiden Wasser und gegebenenfalls die in der Kosmetik üblichen Zusatzstoffe, beispielsweise Parfüm, Verdicker, Farbstoffe, Desodorantien, antimikrobielle Stoffe, rückfettende Agentien, Komplexierungs- und Sequestrierungsagentien, Perlglanzagentien, Pflanzenextrakte, Vitamine, Wirkstoffe und dergleichen.

Die vorliegende Erfindung umfaßt auch ein kosmetisches oder dermatologisches Verfahren zum Schutze der Haut und der Haare vor oxidativen bzw. photooxidativen Prozessen, das dadurch gekennzeichnet ist, daß man ein kosmetisches Mittel, welches eine wirksame Konzentration an erfindungsgemäßen Wirkstoffkombinationen enthält, in ausreichender Menge auf die Haut oder Haare aufbringt.

Die erfindungsgemäßen Wirkstoffe können einzeln oder als Gemisch mehrerer Wirkstoffe verwendet werden und auch einzeln oder als Gemisch in den Zubereitungen enthalten sein.

Als zweckmäßig hat es sich erwiesen, den pH-Wert der erfindungsgemäßen Zubereitungen z.B. im Bereich von 4 bis 9 einzustellen, insbesondere aber im Bereich pH5 bis pH7.

Gegenstand der Erfindung ist auch das Verfahren zur Herstellung der erfindungsgemäßen kosmetischen Mittel, das dadurch gekennzeichnet ist, daß man in an sich bekannter Weise erfindungsgemäße Wirkstoffe in kosmetische und dermatologische Formulierungen einarbeitet. Dies kann z.B. durch Rühren oder Emulgieren geschehen.

Die nachfolgenden Beispiele sollen die vorliegende Erfindung verdeutlichen ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen oder der jeweiligen Mischung bezogen. Die Herstellung der Zubereitungen erfolgt in an sich bekannter Weise.

### Beispiel 1

| W/O Crème | |
|---|---|
| | Gew.-% |
| Paraffinöl | 10 ,00 |
| Petrolatum | 4 ,00 |
| Wollwachsalkohol | 1,00% |
| PEG-7 Hydriertes Rizinusöl | 3,00 |
| Aluminumstearat | 0,40 |
| alpha-Hydroxypalmitinsäure | 0,3 |
| Glycerin | 2,00 |
| Wasser, Konservierungsmittel und Parfüm ad | 100,00 |

### Beispiel 2

| W/O Lotion | |
|---|---|
| | Gew.-% |
| Paraffinöl | 20,00 |
| Petrolatum | 4,00 |
| Glucosesesqiisostearat | 2,00 |
| Aluminumstearat | 0,40 |
| alpha-Hydroxypalmitinsäure | 0,5 |
| Vitamin E Acetat | 2,00 |
| Vitamin C Palmitat | 0,20 |
| Glycerin | 5,00 |
| Wasser, Konservierungsmittel und Parfüm | ad 100,00 |

### Beispiel 3

| O/W Lotion | |
|---|---|
| | Gew.-% |
| Paraffinöl | 8,00 |
| Isopropylpalmitat | 3,00 |
| Petrolatum | 4,00, |
| Cetearylalkohol | 2,00 |
| PEG-40 Rizinusöl | 0,50 |
| Natriumcetearylsulfat | 0,50 |
| Natrium Carbomer | 0,40 |
| alpha-Hydroxypalmitinsäure | 0,2 |
| Glycerin | 3,00 |
| α-Tocopherol | 0,20 |
| Octylmethoxycinnamat | 5,00 |
| Butylmethoxydibenzoylmethan | 1,00 |
| Wasser, Konservierungsmittel und Parfüm | ad 100,00 |

### Beispiel 4

| O/W Crème | |
|---|---|
| | Gew.-% |
| Paraffinöl | 7,00 |
| Avocadoöl | 4,00 |
| Glycerylmonostearat | 2,00 |
| Natriumstearat | 1,00 |
| alpha-Hydroxypalmitinsäure | 0,4 |
| Titandioxid | 1,00 |
| Natriumlactat | 3,00 |
| Glycerin | 3,00 |
| Wasser, Konservierungsmittel und Parfüm | ad 100,00 |

### Beispiel 5

| Lippenpflegestift | |
|---|---|
| | Gew.-% |
| Hydriertes Rizinusöl | 4,00 |
| Ceresin | 8,00 |
| Bienenwachs | 4,00 |
| Carnaubawachs | 2,00 |
| Petrolatum | 40,00 |
| alpha-Hydroxypalmitinsäure | 1,00 |
| β-Carotin | 0,10 |
| Paraffinöl, Pigmente und Farbstoffe | ad 100,00 |

### Beispiel 6

| Liposomenhaltiges Gel | |
|---|---|
| | Gew.-% |
| Lecithin | 6,00 |
| Schibutter | 3,00 |
| alpha-Hydroxypalmitinsäure | 0,1 |
| Vitamin A Palmitat | 0,20 |
| Biotin | 0,08 |
| Natriumcitrat | 0,50 |
| Glycin | 0,20 |
| Harnstoff | 0,20 |
| Natrium PCA | 0,50 |
| Hydrolysiertes Kollagen | 2,00 |
| Xanthan Gummi | 1,40 |
| Sorbitol | 3,00 |
| Wasser, Konservierungsmittel und Parfüm | ad 100,00 |

### Beispiel 7

| Haarkur | |
|---|---|
| | Gew.-% |
| Paraffinöl | 3,00 |
| Mandelöl | 3,00 |
| Cetostearylalcohol | 5,00 |
| PEG - 40 Rizinusöl | 1,00 |
| Natriumcetearylsulfate | 0,50 |
| Sorbitol | 5,00 |
| Glycerin | 5,00 |
| alpha-Hydroxypalmitinsäure | 2,00 |
| Dilaurylthiodipropionat | 0,05 |
| Wasser, Konservierungsmittel und Parfüm | ad 100,00 |

### Beispiel 8

| Massagecrème | |
|---|---|
| | Gew.-% |
| Stearylalkohol | 2,00 |
| Petrolatum | 4,00 |
| Dimethicon | 2,00 |
| Isopropylpalmitat | 6,00 |
| Cetearylalkohol | 4,00 |
| PEG- 40 Hydriertes Rizinusöl | 2,00 |
| alpha-Hydroxypalmitinsäure | 0,3 |
| Glycerin | 3,00 |
| Wasser, Konservierungsmittel und Parfüm | ad 100,00 |

## Patentansprüche

1. Verwendung von einer alpha-Hydroxyfettsäure oder mehreren alpha-Hydroxyfettsäuren oder Salzen davon zur Prophylaxe und Behandlung von schädigenden oxidativen Einwirkungen auf die Haut oder die Haare.

2. Verwendung von kosmetischen oder dermatologischen Zubereitungen mit einem Gehalt an einer alpha-Hydroxyfettsäure oder mehreren alpha-Hydroxyfettsäuren oder Salzen davon zur Prophylaxe und Behandlung von schädigenden oxidativen Einwirkungen auf die Haut oder die Haare.

3. Verwendung gemäß Anspruch 1, dadurch gekennzeichnet, daß die alpha-Hydroxyfettsäure alpha-Hydroxypalmitinsäure ist.

4. Verwendung gemäß Anspruch 2, dadurch gekennzeichnet, daß die Zubereitungen 0,001 bis 10 Gew.-%, bevorzugt 0,1 Gew.-% bis 1 Gew.-%, insbesondere aber 0,1 Gew.-% bis 0,5 Gew.-%, jeweils bezogen auf das Gesamtgewicht der Zubereitungen, an den erfindungsgemäßen Wirkstoffen enthalten.

5. Verwendung gemäß Anspruch 1 oder 2, dadurch gekennzeichnet, daß die alpha-Hydroxyfettsäuren mit Antioxidantien kombiniert werden.

6. Verwendung gemäß Anspruch 1 oder 2, dadurch gekennzeichnet, daß die alpha-Hydroxyfettsäuren mit mindestens einem UVA-Filter und/oder mindestens einem UVB-Filter kombiniert werden.
